# EUROPEAN PATENT APPLICATION

(11) **EP 2 486 846 A1**
(43) Date of publication of application: **15.08.2012**
(21) Application number: 10821905.6
(22) Date of filing: 29.09.2010
(51) Int. Cl.: A61B 1/04, A61B 1/00

(54) **ENDOSCOPE DEVICE**

(30) Priority: 09.10.2009 JP 2009235411
(71) Applicant: Olympus Medical Systems Corp., Tokyo 151-0072 (JP)
(72) Inventor: YOSHIE, Michifumi, Tokyo 192-8512 (JP); KUSHIDA, Atsuhiko, Tokyo 192-8512 (JP)
(74) Representative: Schicker, Silvia
(86) International application number: PCT/JP2010/066980
(87) International publication number: WO 2011/043234

(57) **Abstract**

An endoscopic device includes an endoscope (10) provided with a distal hard portion (14) on a most distal direction side thereof, a manipulator (50) which is configured to be inserted through an insertion passage (42) provided in the endoscope (10) or in a tube (95) separated from the endoscope (10), and an imaging element (30) which is provided in the distal hard portion (14) of the endoscope (10) and which is configured to image a subject and a distal end portion of the manipulator (50). The endoscopic device includes a marking portion (70A-70D) provided at the distal end portion of the manipulator (50) and within an imaging range of the imaging element (30), the position of the marking portion (70A-70D) changing in a direction around an axis in response to a rolling motion of the manipulator (50) relative to the endoscope (10).

## Description

### Technical Field

The present invention relates to an endoscopic device including an endoscope, and a manipulator which is configured to be inserted through a treatment tool insertion passage provided in the endoscope or in a tube separated from the endoscope.

### Background Art

There has been generally used as an endoscopic device wherein an endoscope is inserted into a body cavity, and a manipulator is protruded from a distal end of the endoscope or a tubular component separate from the endoscope via a treatment insertion passage of the endoscope or the tubular component. In the endoscopic device, the manipulator performs a treatment in the body cavity under the endoscopic observation.

For accurate manipulation to perform curving motion or gripping motion of the manipulator during a treatment, it is important for an operator to recognize the amount of rolling of a distal end of the manipulator in a direction around the axis. In the conventional endoscopic device, a detector is provided at a proximal end portion of the manipulator, and the amount of rolling of the proximal end portion of the manipulator in a direction around the axis is detected by the detector. The rolling amount at the proximal end portion of the manipulator is then determined as the amount of rolling at the distal end portion of the manipulator in the direction around the axis.

Patent Literature 1 has disclosed an endoscopic device including a grip forceps to be inserted through a forceps channel of an endoscope. In this endoscopic device, a roller that rolls in response to the back-and-forth motion of the grip forceps is provided at a proximal end portion of the forceps channel. A rolling angle of the roller is detected by a detector, and an amount of back-and-forth motion of the grip forceps in the longitudinal direction is calculated on the basis of the rolling angle.

Patent Literature 2 has disclosed a robotic surgery system including a rigid manipulator supporting an end effecter which is a treatment tool, a rigid linkage supporting the manipulator, and an imaging system. The linkage is composed of arms joined by joints, and the joints of the linkage are actuated by a servomechanism. This robot surgical system also has a camera coordinate system having its origin in an imaging element of the imaging system, and a manipulator coordinate system having its origin in a distal end of the manipulator. The manipulator coordinate system changes with the actuation of the joints of the linkage. In this case, the amount of rolling in a direction around the axis of the manipulator is calculated on the basis of the operation amount of the joints detected by a sensor system connected to the linkage. On the basis of the rolling amount of the manipulator, a relation between the camera coordinate system and the manipulator coordinate system is found. When performing a treatment, an operator inputs an instruction from a controller with reference to the camera coordinate system. However, as the manipulator is operated in accordance with the manipulator coordinate system, the relation between the camera coordinate system and the manipulator coordinate system has to be considered. If the operator performs manipulation with reference to the camera coordinate system without considering the relation between the camera coordinate system and the manipulator coordinate system, the manipulator may move in a direction different from a direction intended by the operator. Therefore, in this robot surgical system, the servomechanism converts the instruction input based on the camera coordinate system from the controller to an instruction based on the manipulator coordinate system in accordance with the relation between the camera coordinate system and the manipulator coordinate system. As a result of this conversion, the operator can move the manipulator in an intended direction without considering the relation between the camera coordinate system and the manipulator coordinate system.

### Citation List

### Patent Literature

Patent Literature 1: Jpn. Pat. Appln. KOKAI Publication No. 2008-212349
Patent Literature 2: The specification of US Patent No. 6441577

### Summary of Invention

### Technical Problem

However, an actually-used manipulator is provided with an elongated and flexible manipulator insertion portion. In addition, in the state where the manipulator is inserted in the treatment tool insertion passage, friction is caused between the manipulator and the inner wall of the treatment tool insertion passage. Therefore, there may be a case where the torque of the manipulator is not sufficiently transmitted from the proximal end portion to the distal portion of the manipulator. In such a case, the amount of rolling in the direction around the axis differs between the proximal end portion of the manipulator and the distal end portion of the manipulator. Therefore, the structure of the conventional endoscopic device has problems in that the operator cannot accurately recognize the amount of rolling at the distal end portion of the manipulator.

A detector configured to detect the amount of rolling may be arranged at the distal end portion of the manipulator. If this is done, however, the structure of the distal end portion of the manipulator is inevitably complex, and the diameter of the manipulator is large, accordingly.

Patent literature 1 mentioned above may disclose a structure to calculate the amount of back-and-forth motion of the grip forceps in the longitudinal direction. However, patent literature 1 does not disclose any structure to calculate the amount of rolling of the distal end portion of the grip forceps in the direction around the axis.

According to patent literature 2, the arms of the linkage and the manipulator are rigid. Even when the manipulator is rolled by the actuation of the joints of the linkage, the amount of rolling at the proximal end portion of the manipulator and the amount of rolling at the distal end portion of the manipulator are the same. Therefore, the amount of rolling at the distal end portion of the manipulator can be calculated by detecting the amount of actuation of the joints of the linkage. However, in the manipulator used in the endoscopic device of flexible endoscope type, the amount of rolling differs between the proximal end portion and the distal end portion. Therefore, the amount of rolling at the distal end portion of the manipulator cannot be accurately calculated based on the amount of rolling at the proximal end portion of the manipulator. Even if the mechanism of converting the instruction input disclosed in patent literature 2 is applied to the endoscopic device of flexible endoscope type, the relationships between the camera coordinate system and the manipulator coordinate system cannot be obtained, and conversion of the instruction input from the controller cannot be performed.

The present invention has been developed in view of the above circumstances, and an object thereof is to provide an endoscopic device capable of accurately calculating the amount of rolling at the distal end portion of a manipulator without having to provide a detector at the distal end portion of the manipulator.

### Solution to Problem

To solve above mentioned problems, according to one aspect of the invention, an endoscopic device includes an endoscope provided with a distal hard portion on a most distal direction side thereof; a manipulator which is configured to be inserted through an insertion passage provided in the endoscope or in a tube separated from the endoscope; an imaging element which is provided in the distal hard portion of the endoscope and which is configured to image a subject and a distal end portion of the manipulator; and a marking portion provided at the distal end portion of the manipulator and within an imaging range of the imaging element, the position of the marking portion changing in a direction around an axis in response to a rolling motion of the manipulator relative to the endoscope.

It is preferable that the endoscopic device further includes an image processor which is configured to process an image signal of an observation image captured by the imaging element; and a calculation unit which is configured to calculate a rolling amount of the manipulator relative to the endoscope in the direction around the axis on the basis of the image signal from the image processor. Further, it is preferable that the calculation unit includes a recorder in which known information is recorded, a distortion remover which is configured to remove distortion from the observation image, a marking extractor which is configured to extract position information and posture information of the marking portion on the distortion-free observation image, and a roll information calculator which is configured to calculate the rolling amount of the manipulator relative to the endoscope in the direction around the axis on the basis of the extracted position information and posture information of the marking portion on the observation image and the known information recorded in the recorder. Further, it is preferable that the endoscopic device further includes an instruction input unit which configured to input an instruction based on a camera coordinate system having its origin in the imaging element on the observation image to manipulate the manipulator, and that the calculation unit includes an input instruction converter, the input instruction converter being configured to find a relation between the camera coordinate system and a manipulator coordinate system on the basis of the rolling amount of the manipulator relative to the endoscope calculated by the roll information calculator, and being configured to convert the instruction based on the camera coordinate system from the instruction input unit to an instruction based on the manipulator coordinate system in accordance with the relation between the camera coordinate system and the manipulator coordinate system, and the manipulator coordinate system having its origin in a distal end of the manipulator on the observation image.

Further, the marking portion may include belt-shaped portions which extend in a longitudinal direction and which are provided with colors different from that of the manipulator and different from one another. Also, the marking portion may include belt-shaped portions which extend in a longitudinal direction and which are provided with pattern designs different from one another.

### Advantageous Effects of Invention

According to the present invention, an endoscopic device, capable of accurately calculating the amount of rolling at the distal end portion of a manipulator without having to provide a detector at the distal end portion of the manipulator, can be provided.

### Brief description of Drawings

FIG. 1 is a perspective view showing a system that uses an endoscopic device according to a first embodiment of the present invention;
FIG. 2A is a block diagram showing the endoscopic device according to the first embodiment;
FIG. 2B is a block diagram showing the configuration of a motor unit of the endoscopic device according to the first embodiment;
FIG. 3 is a perspective view showing the configuration of a distal end portion of an endoscope and a distal end portion of a manipulator of the endoscopic device according to the first embodiment;
FIG. 4 is a perspective view showing the configuration of the distal end portion of the manipulator of the endoscopic device according to the first embodiment;
FIG. 5 is a schematic diagram showing the configuration of the distal end portion of the manipulator of the endoscopic device according to the first embodiment;
FIG. 6 is a sectional view showing the configuration of a third curving piece of the manipulator according to the first embodiment;
FIG. 7 is a block diagram showing the configuration of a calculation unit of the endoscopic device according to the first embodiment;
FIG. 8 is a flowchart showing a method of calculating a rolling amount of the distal end portion of the manipulator from an observation image through the endoscope, in the endoscopic device according to the first embodiment;
FIG. 9A is a schematic diagram showing an observation image of the endoscopic device according to the first embodiment in a particular condition;
FIG. 9B is a schematic diagram showing an observation image of a condition in which the manipulator is rolled substantially 90° relative to the endoscope and a first joint of a manipulator curving portion is curved from the condition of FIG. 9A;
FIG. 10 is a block diagram showing the configuration of a calculation unit of an endoscopic device according to a second embodiment of the present invention;
FIG. 11A is a schematic diagram showing an observation image of the endoscopic device according to the second embodiment in an unrolled condition;
FIG. 11B is a schematic diagram showing an observation image of a condition in which a distal end portion of a manipulator is rolled substantially 90° relative to an endoscope in a direction around the axis from the unrolled condition of FIG. 11A;
FIG. 12 is a flowchart showing a method of converting an input instruction from an operator by an input instruction converter of the endoscopic device according to the second embodiment;
FIG. 13 is a sectional view showing the configuration of a third curving piece of a manipulator according to a modification of the present invention; and
FIG. 14 is a perspective view showing the configuration of a distal end of an endoscope and a distal end of a manipulator of an endoscopic device according to another modification of the present invention.

### Description of Embodiments

### (First Embodiment)

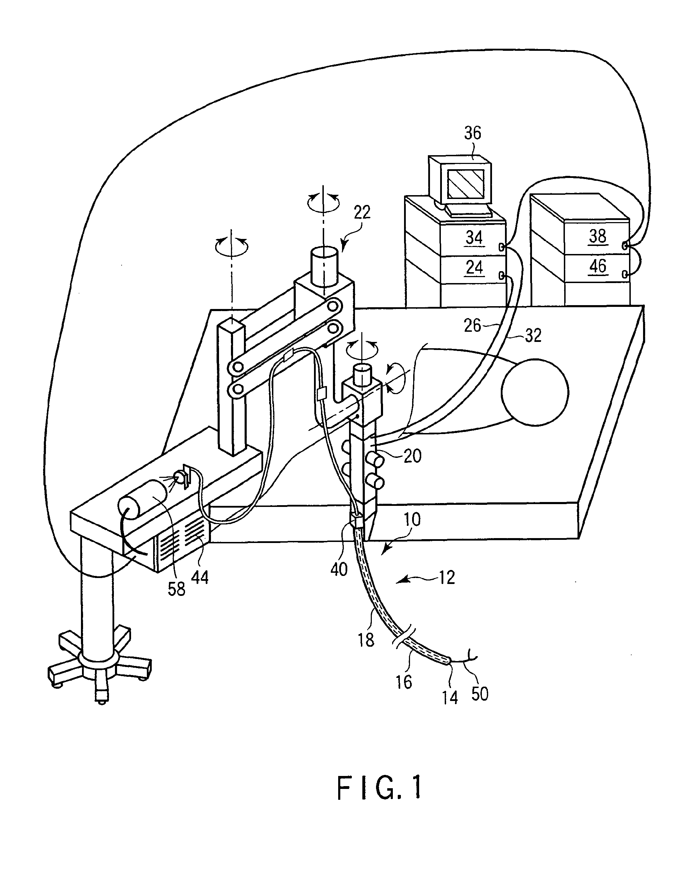

A first embodiment of the present invention is described with reference to FIG. 1 to FIG. 9B.

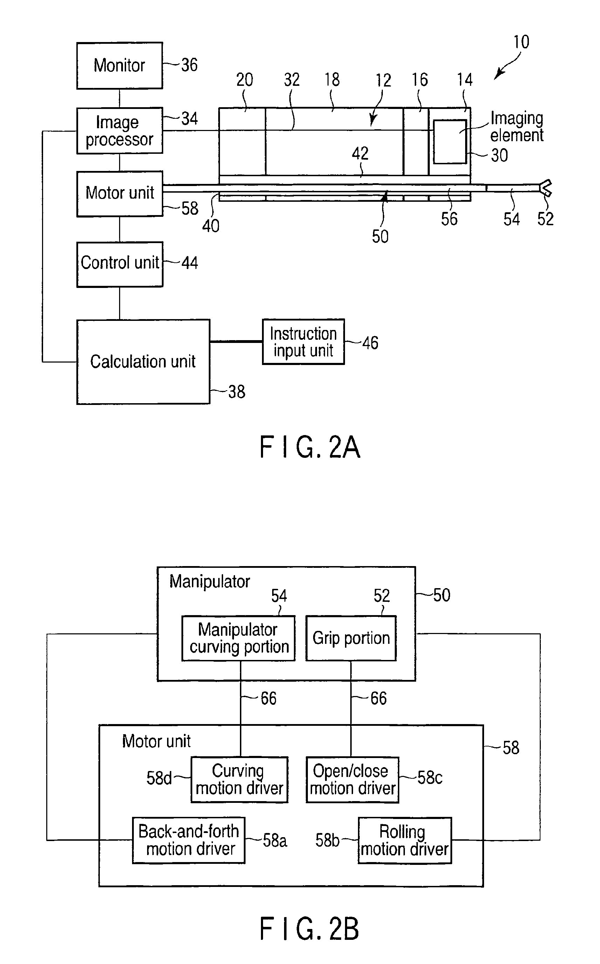

FIG. 1 is a diagram showing a system that uses an endoscopic device. FIG. 2A is a diagram showing the endoscopic device. As shown in FIG. 1, an active endoscope 10 (hereinafter simply referred to as an endoscope 10) of the endoscopic device includes an endoscopic insertion portion 12 to be inserted into a body cavity. The endoscopic insertion portion 12 is provided with, from a distal direction side in order, a distal hard portion 14 provided at a most distal direction side, an endoscopic curving portion 16 to be curved, and an elongated and flexible endoscopic flexible tubular portion 18. An endoscopic operation portion 20 is coupled to a proximal direction side of the endoscopic insertion portion 12. The endoscopic operation portion 20 is removably attached to a movable endoscopic stand 22. The endoscopic operation portion 20 can be moved to and fixed at any position by the movable endoscopic stand 22.

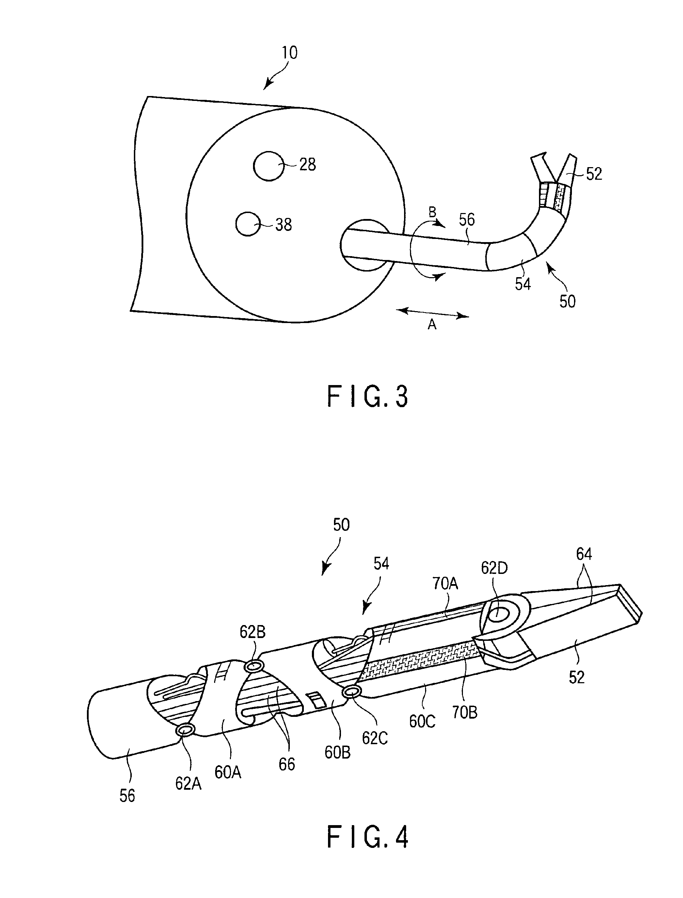

As shown in FIG. 1, the endoscopic device includes a light source unit 24. A light guide 26 is connected to the light source unit 24. The light guide 26 extends to the distal hard portion 14 through the endoscopic operation portion 20, the endoscopic flexible tubular portion 18, and the endoscopic curving portion 16. Light emitted from the light source unit 24 is guided to the distal hard portion 14 by the light guide 26, and irradiated to a subject from an illumination window 28 (see FIG. 3) provided in the distal face of the distal hard portion 14.

As shown in FIG. 2A, the distal hard portion 14 of the endoscopic insertion portion 12 includes therein an imaging element 30 which images the subject. An imaging cable 32 is connected to the imaging element 30. The imaging cable 32 is connected to an image processor 34 through the endoscopic curving portion 16, the endoscopic flexible tubular portion 18, and the endoscopic operation portion 20. The image processor 34 is provided outside the endoscope 10. The image processor 34 is connected to a monitor 36 which is a display section, and a calculation unit 38. An observation image captured by the imaging element 30 through an observation window 37 (see FIG. 3) is converted to an image signal and output to the image processor 34. The observation image is processed in the image processor 34, and the observation image is displayed on the monitor 36. The image processor 34 also outputs, to the calculation unit 38, image data input as the image signal.

As shown in FIG. 2A, the endoscopic operation portion 20 of the endoscope 10 is provided with a treatment tool insertion opening 40. A treatment tool channel 42 which is a treatment tool insertion passage extends to the distal hard portion 14 from the treatment tool insertion opening 40. A manipulator 50 which is a treatment tool is inserted through the treatment tool channel 42 of the endoscope 10 to be movable back and forth in a longitudinal direction. The manipulator 50 is connected to a motor unit 58 provided on the movable endoscopic stand 22 (see FIG. 1). The motor unit 58 is connected to a control unit 44 which drives and controls the motor unit 58. The control unit 44 is connected to the calculation unit 38, and the calculation unit 38 is connected to an instruction input unit 46. The control unit 44 drives and controls the motor unit 58 in accordance with an instruction input in the instruction input unit 46 and in accordance with a calculation result in the calculation unit 38.

As shown in FIG. 2A, the manipulator 50 is provided with, from the distal direction side in order, a grip portion 52 to be opened/closed, a manipulator curving portion 54 to be curved, and an elongated and flexible manipulator insertion portion 56. The manipulator insertion portion 56 extends to the motor unit 58 toward a proximal direction. As shown in FIG. 2B, the motor unit 58 includes a back-and-forth motion driver 58a such as a motor which is a driving source of the back-and-forth motion of the manipulator 50, and a rolling motion driver 58b such as a motor which is a driving source of the rolling motion of the manipulator 50. When the back-and-forth motion driver 58a of the motor unit 58 is driven, the manipulator insertion portion 56 moves back and forth in the longitudinal direction (an arrow A in FIG. 3). When the rolling motion driver 58b of the motor unit 58 is driven, the manipulator insertion portion 56 rolls in a direction around the axis of the manipulator 50 (an arrow B in FIG. 3). In this way, the back-and-forth motion and rolling motion of the manipulator 50 are achieved. The motor unit 58 also includes an encoder (not shown) which detects the amount of the back-and-forth motion of the manipulator 50 in the longitudinal direction.

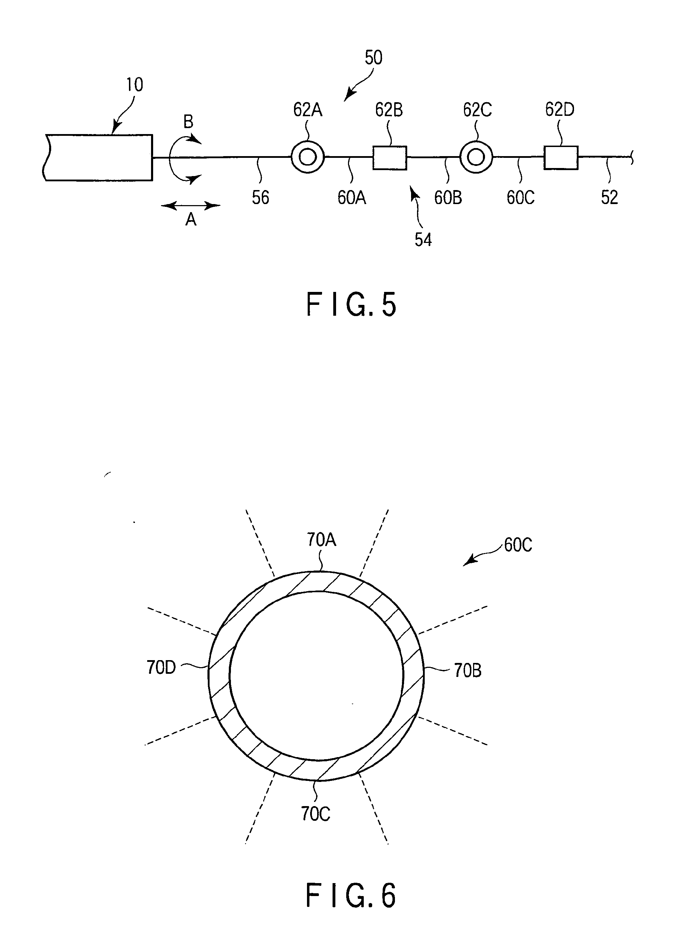

FIG. 4 and FIG. 5 are diagrams showing the configuration of a distal end portion of the manipulator 50. As shown in FIG. 4 and FIG. 5, the manipulator curving portion 54 includes three curving pieces 60A to 60C. The first curving piece 60A disposed on the most proximal direction side among the three curving pieces 60A to 60C is substantially coaxially coupled to the manipulator insertion portion 56 via a first joint 62A. The second curving piece 60B is substantially coaxially coupled to the distal direction side of the first curving piece 60A via a second joint 62B. Similarly, the third curving piece 60C is substantially coaxially coupled to the distal direction side of the second curving piece 60B via a third joint 62C, and the grip portion 52 is substantially coaxially coupled to the distal direction side of the third curving piece 60C via a fourth joint 62D. The first curving piece 60A is rotatable around the rotation axis of the first joint 62A relative to the manipulator insertion portion 56. The first curving piece 60A and the second curving piece 60B are rotatable relative to each other around the rotation axis of the second joint 62B. Similarly, the second curving piece 60B and the third curving piece 60C are rotatable relative to each other around the rotation axis of the third joint 62C, and the third curving piece 60C and the grip portion 52 are rotatable relative to each other around the rotation axis of the fourth joint 62D. In the grip portion 52, a pair of jaws 64 is capable of opening/closing about the rotation axis of the fourth joint 62D. The rotation axes of the first joint 62A and the third joint 62C are substantially perpendicular to the axis of the manipulator 50. The rotation axes of the second joint 62B and the fourth joint 62D are substantially perpendicular to the axis of the manipulator 50 and also substantially perpendicular to the rotation axes of the first joint 62A and the third joint 62C. The rotation axes of the first joint 62A and the third joint 62C are substantially perpendicular to the rotation axes of the second joint 62B and the fourth joint 62D such that the curving directions of the first joint 62A and the third joint 62C are substantially perpendicular to the curving directions of the second joint 62B and the fourth joint 62D. Thus, the manipulator curving portion 54 is a curving portion having two degrees of freedom.

As shown in FIG. 4, operation wires 66 are connected to the grip portion 52. Each of the operation wires 66 is used to open/close the grip portion 52 or to curve the manipulator curving portion 54. Each of the operation wires 66 is connected to the motor unit 58 through the manipulator insertion portion 56. As shown in FIG. 2B, the motor unit 58 includes an open/close motion driver 58c which is a driving source of the open/close motion of the grip portion 52, and a curving motion driver 58d which is a driving source of the curving motion of the manipulator curving portion 54. The open/close motion driver 58c includes motors and pulleys. The open/close motion driver 58c is driven such that the operation wires 66 which opens/closes the grip portion 52 moves in the longitudinal direction, and the jaws 64 of the grip portion 52 open/close. The curving motion driver 58d includes motors and pulleys. The curving motion driver 58d is driven such that the operation wires 66 used of curving motion moves in the longitudinal direction, and each of the first to fourth joints 62A to 62D rotates around the rotation axis. Thus, the manipulator curving portion 54 performs the curving motion. The motor unit 58 also includes an encoder (not shown) which detects the amount of the movement of each of the operation wires 66 in the longitudinal direction. A detection result in the encoder is output to the calculation unit 38. The calculation unit 38 calculates the open/close motion amount of the grip portion 52 and the rotation operation amount of each of the first to fourth joints 62A to 62D in accordance with the detection result in the encoder.

FIG. 6 is a diagram showing the configuration of the third curving piece 60C. As shown in FIG. 4 and FIG. 6, (in the present embodiment, four) belt-shaped marking portions 70A to 70D extending in the longitudinal direction are provided on the outer peripheral surface of the third curving piece 60C of the manipulator curving portion 54. A color different from the color of the manipulator 50 is assigned to each of the marking portions 70A to 70D. The colors of the marking portions 70A to 70D are different from one another. For example, blue is assigned to the first marking portion 70A, yellow is assigned to the second marking portion 70B, green is assigned to the third marking portion 70C, and black is assigned to the fourth marking portion 70D. That is, the marking portions 70A to 70D are belt-shaped portions which extend in the longitudinal direction and which are provided with colors different from one another. The marking portions 70A to 70D are separate from one another in the direction around the axis of the manipulator 50, and are arranged substantially 90° apart from one another in the direction around the axis of the manipulator 50. The position of each of the marking portions 70A to 70D in the direction around the axis of the manipulator 50 changes with the rolling motion of the manipulator 50.

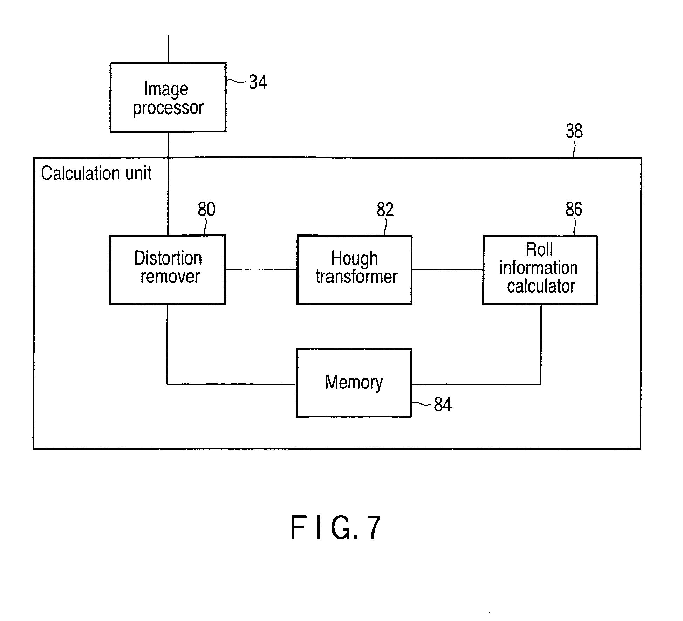

FIG. 7 is a diagram showing the configuration of the calculation unit 38. As shown in FIG. 7, the calculation unit 38 includes a distortion remover 80, a Hough transformer 82, a memory 84 which is a recorder, and a roll information calculator 86. The distortion remover 80 is connected to the Hough transformer 82 and the memory 84. The Hough transformer 82 and the memory 84 are connected to the roll information calculator 86.

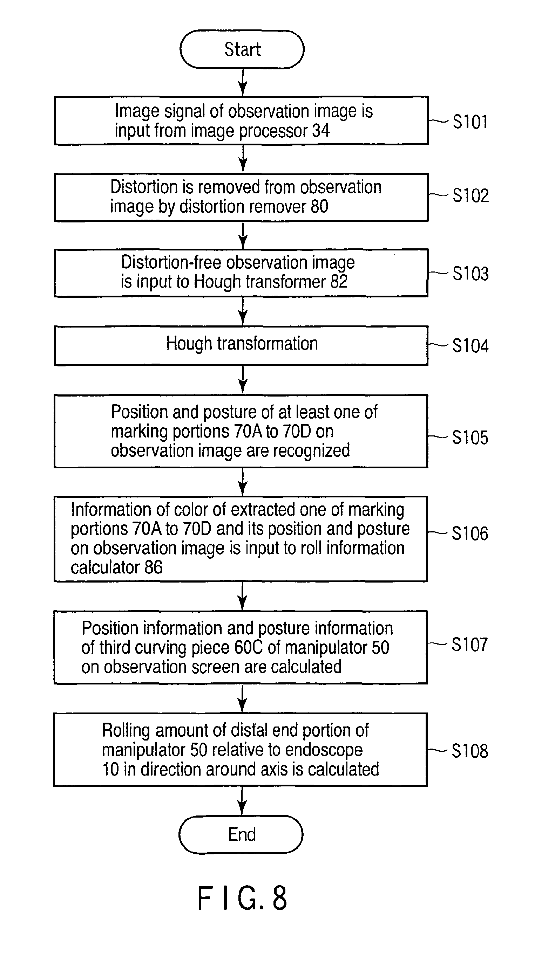

Now, a method of calculating a rolling amount of the distal end portion of the manipulator 50 from an observation image through the endoscope 10 is described with reference to FIG. 8 to FIG. 9B. As shown in FIG. 8, an image signal of the observation image is input to the distortion remover 80 from the image processor 34 at the start of operation (step S101). Distortion information of the observation image is recorded in the memory 84. The distortion remover 80 removes distortion from the observation image on the basis of the distortion information from the memory 84 (step S102).

The distortion-free observation image is input to the Hough transformer 82 (step S103). The Hough transformer 82 subjects the distortion-free observation image to Hough transformation (step S104). The Hough transformation is a method of extracting the positions and postures, in the observation image, of the marking portions 70A to 70D presented in the observation image. That is, the Hough transformation is used to recognize where the belt-shaped marking portions 70A to 70D marked with the particular colors are located in the observation image in what posture. The specific method of the Hough transformation is not described in detail because the description-is found in Reference document 1 (Duda, R. O. and P. E. Hart, "Use of the Hough Transformation to Detect Lines and Curves in Pictures," Comm. ACM, January, 1972, Vol. 15, pp. 11 - pp. 15).

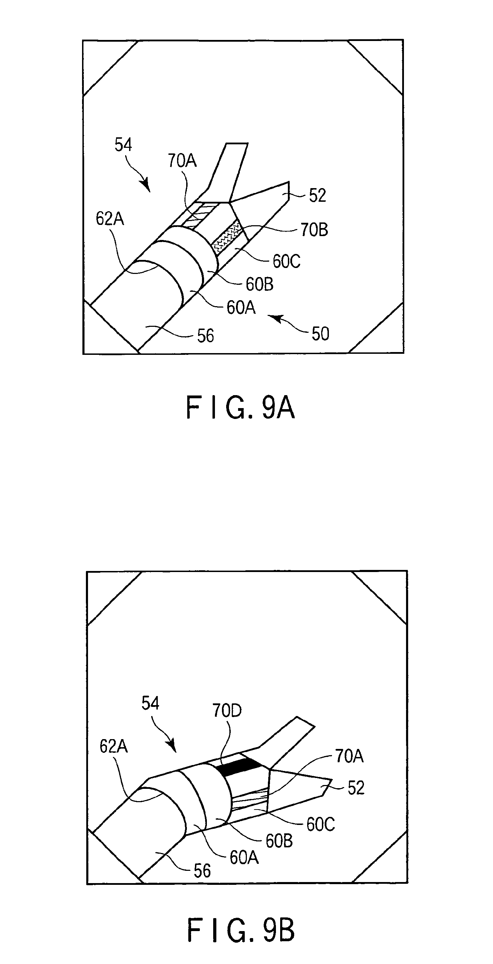

The marking portions 70A to 70D are separate from one another in the direction around the axis of the manipulator 50, and are arranged substantially 90° apart from one another in the direction around the axis of the manipulator 50. FIG. 9A shows an observation image displayed on the monitor 36 in a particular condition. FIG. 9B shows an observation image of a condition in which the manipulator 50 is rolled substantially 90° relative to the endoscope 10 and the first joint 62A of the manipulator curving portion 54 is curved from the condition of FIG. 9A. The marking portions 70A to 70D are arranged as described above, so that the dimensions of at least one of the marking portions 70A to 70D in the direction around the axis of the manipulator 50 can be recognized on an observation screen in any condition as shown in FIG. 9A and FIG. 9B. That is, the belt shape of at least one of the marking portions 70A to 70D can be recognized on the observation screen regardless of, for example, the angle of view of the imaging element 30, the positional relation between the imaging element 30 and the manipulator 50, the curving motion amount of the manipulator 50, and the rolling motion amount of the manipulator 50. Thus, the position and posture of at least one of the marking portions 70A to 70D on the observation image are recognized by the Hough transformation (step S105). That is, the Hough transformer 82 serves as a marking extractor which extracts the position and posture of at least one of the marking portions 70A to 70D on the observation image. Information on the color of at least one of the marking portions 70A to 70D and on its position and posture on the observation screen extracted by the Hough transformation is input to the roll information calculator 86 (step S106).

Known information such as position, dimension and color information of the marking portions 70A to 70D in the manipulator 50 and view angle information of the imaging element 30 is recorded in the memory 84. On the basis of the information input from the Hough transformer 82 and the known information recorded in the memory 84, the roll information calculator 86 calculates position information and posture information of the third curving piece 60C of the manipulator 50 on the observation screen (step S107). On the basis of the calculated position information and posture information of the third curving piece 60C, a rolling amount of the distal end portion of the manipulator 50 relative to the endoscope 10 in the direction around the axis is calculated (step S108).

Now, the function of the endoscopic device according to the present embodiment is described. When the operator performs a treatment using the endoscopic device, the operator inserts the manipulator 50 through the treatment tool channel 42 of the endoscope 10. The motor unit 58 then causes the manipulator 50 to perform the back-and-forth motion, rolling motion, curving motion, and gripping motion to treat an affected part. In this case, light emitted from the light source unit 24 is guided to the distal hard portion 14 of the endoscope 10 by the light guide 26, and irradiated to the subject from the illumination window 28 of the distal hard portion 14. The subject is then imaged by the imaging element 30 provided in the distal hard portion 14 through the observation window 37, and an image signal is output to the image processor 34. The output image signal is processed in the image processor 34, and an observation image is displayed on the monitor 36. The observation image on the monitor 36 shows the affected part and the condition of the distal end portion of the manipulator 50. The operator manipulates the manipulator 50 while viewing the displayed observation image, and thereby treats the affected part.

The third curving piece 60C of the manipulator curving portion 54 of the manipulator 50 is provided with the marking portions 70A to 70D. On the observation image, the dimensions of at least one of the marking portions 70A to 70D in the direction around the axis of the manipulator 50 can be recognized. When the manipulator 50 is in an unrolled condition and not rolled relative to the endoscope 10 in the direction around the axis, the operator recognizes the position of each of the marking portions 70A to 70D relative to the endoscope 10 in the direction around the axis. Thus, in accordance with the information of the marking portions 70A to 70D on the observation image and the position information of the marking portions 70A to 70D in the unrolled condition, the operator can recognize the rough rolling amount of the distal end portion of the manipulator 50 relative to the endoscope 10 in the direction around the axis.

The image signal output to the image processor 34 is input to the distortion remover 80 of the calculation unit 38. The distortion remover 80 removes distortion from the observation image on the basis of the distortion information from the memory 84. The Hough transformer 82 then subjects the distortion-free observation image to Hough transformation. As a result of the Hough transformation, the position and posture of at least one of the marking portions 70A to 70D on the observation image are recognized as described above. Information of the marking color of at least one of the marking portions 70A to 70D and its position and posture on the observation image extracted by the Hough transformation is input to the roll information calculator 86. On the basis of the information input from the Hough transformer 82 and the known information recorded in the memory 84, the roll information calculator 86 calculates a rolling amount of the distal end portion of the manipulator 50 relative to the endoscope 10 in the direction around the axis as described above. In this way, the rolling amount of the distal end portion of the manipulator 50 in the direction around the axis can be accurately calculated without providing a detector at the distal end portion of the manipulator 50.

Thus, the endoscopic device having the above-described configuration has the following advantages. That is, in the endoscopic device according to the present embodiment, the third curving piece 60C of the manipulator curving portion 54 of the manipulator 50 is provided with the marking portions 70A to 70D. On the observation image, the dimensions of at least one of the marking portions 70A to 70D in the direction around the axis of the manipulator 50 can be recognized. When the manipulator 50 is in an unrolled condition and not rolled relative to the endoscope 10 in the direction around the axis, the operator recognizes the position of each of the marking portions 70A to 70D relative to the endoscope 10 in the direction around the axis. Thus, in accordance with the information of the marking portions 70A to 70D on the observation image and the position information of the marking portions 70A to 70D in the unrolled condition, the operator can recognize the rough rolling amount of the distal end portion of the manipulator 50 relative to the endoscope 10 in the direction around the axis.

Furthermore, in the endoscopic device according to the present embodiment, the image signal is input to the distortion remover 80 of the calculation unit 38 from the image processor 34. The distortion remover 80 removes distortion from the observation image on the basis of the distortion information from the memory 84. The Hough transformer 82 then subjects the distortion-free observation image to Hough transformation. As a result of the Hough transformation, the position and posture of at least one of the marking portions 70A to 70D on the observation image are recognized. Information of the marking color of at least one of the marking portions 70A to 70D and its position and posture on the observation image extracted by the Hough transformation is input to the roll information calculator 86. On the basis of the information input from the Hough transformer 82 and the known information recorded in the memory 84, the roll information calculator 86 calculates a rolling amount of the distal end portion of the manipulator 50 relative to the endoscope 10 in the direction around the axis. In this way, the rolling amount of the distal end portion of the manipulator 50 in the direction around the axis can be accurately calculated without providing a detector at the distal end portion of the manipulator 50.

### (Second Embodiment)

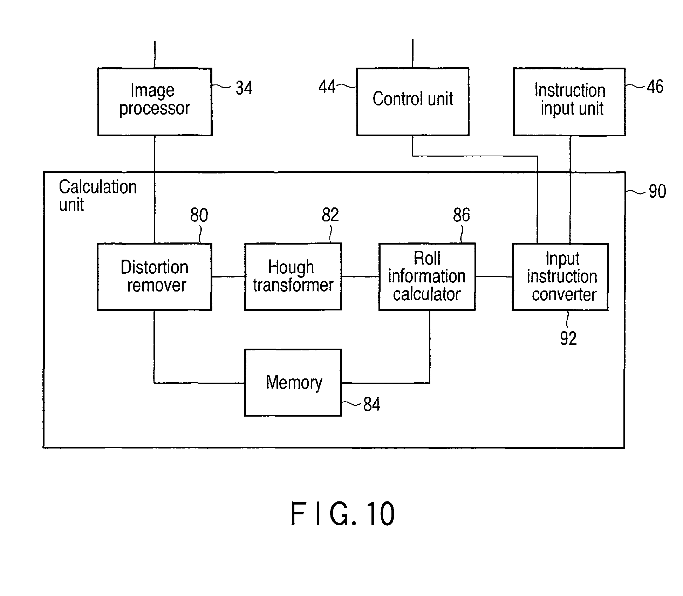

A second embodiment of the present invention is described next with reference to FIG. 10 to FIG. 12. In the present embodiment, the configuration according to the first embodiment is modified as follows. The same signs are properly assigned to the components having the same functions as components according to the first embodiment, and these components are not described in detail.

FIG. 10 is a diagram showing the configuration of a calculation unit 90 according to the present embodiment. As shown in FIG. 10, the calculation unit 90 includes a distortion remover 80, a Hough transformer 82, a memory 84, and a roll information calculator 86 similarly to the calculation unit 38 according to the first embodiment. The roll information calculator 86 is connected to an input instruction converter 92 provided in the calculation unit 90. The input instruction converter 92 is connected to a control unit 44 and an instruction input unit 46.

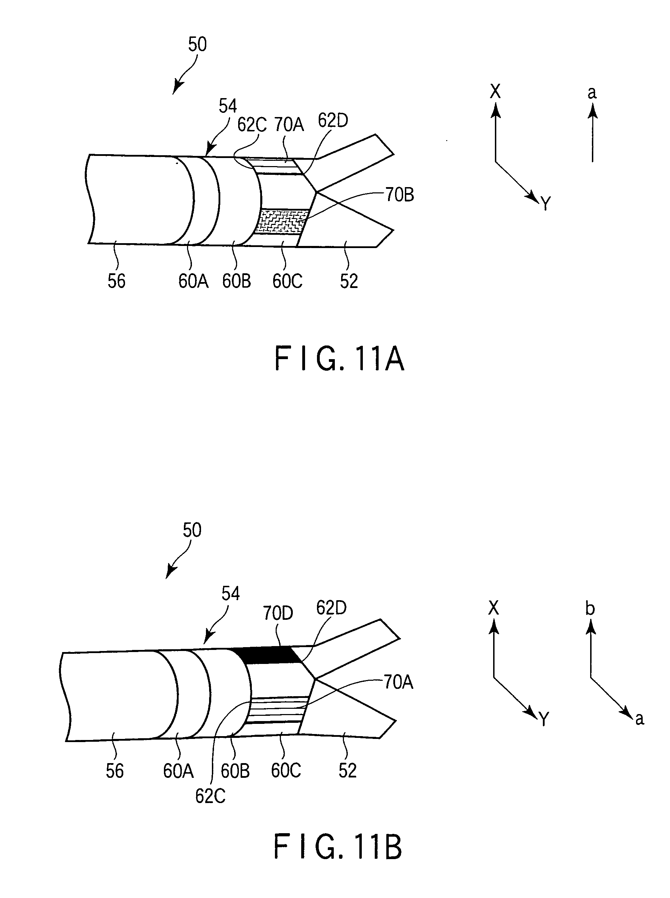

The input instruction converter 92 is described below in detail with reference to FIG. 11A to FIG. 12. On an observation image displayed on a monitor 36, there are a camera coordinate system having its origin in an imaging element 33 of a distal hard portion 14 of an endoscope 10, and a manipulator coordinate system having its origin in a grip portion 52 at a distal end of a manipulator 50. The camera coordinate system is changed by the rotation of the endoscope 10 of the imaging element 33 in a direction around the axis, that is, by rolling the endoscope 10. On the other hand, the manipulator coordinate system is changed by rolling the manipulator 50. Therefore, if the manipulator 50 is rolled relative to the endoscope 10, the relation between the camera coordinate system and the manipulator coordinate system changes.

For example, when the manipulator 50 is in an unrolled condition and not rolled relative to the endoscope 10 in the direction around the axis, an observation image shown in FIG. 11A is displayed on the monitor 36. Here, if the upward direction on the observation image is an X-direction of the camera coordinate system, the X-direction of the camera coordinate system substantially corresponds to an a-direction of the manipulator coordinate system. The a-direction of the manipulator coordinate system substantially corresponds to the direction in which a first marking portion 70A is disposed when viewed from the central axis of a third curving piece 60C of the manipulator 50. In general, the manipulator 50 is manipulated in accordance with the manipulator coordinate system. Thus, in order to curve the manipulator 50 in the X-direction of the camera coordinate system in the condition of FIG. 11A, an instruction to curve, for example, a fourth joint 62D in the a-direction of the manipulator coordinate system has to be input to the instruction input unit 46.

If the distal end portion of the manipulator 50 is rolled substantially 90° relative to the endoscope 10 in the direction around the axis from the unrolled condition of FIG. 11A, an observation image shown in FIG. 11B is displayed on the monitor 36. In this case, the X-direction of the camera coordinate system substantially corresponds to a b-direction of the manipulator coordinate system. The b-direction of the manipulator coordinate system is substantially perpendicular to the a-direction, and substantially corresponds to the direction in which a fourth marking portion 70D is disposed when viewed from the central axis of the third curving piece 60C of the manipulator 50. In general, input from the instruction input unit 46 and the manipulation of the manipulator 50 are based on the manipulator coordinate system. Thus, in order to curve the manipulator 50 in the X-direction of the camera coordinate system in the condition of FIG. 11B, an instruction to curve, in the b-direction of the manipulator coordinate system, for example, a third joint 62C which curves in a direction substantially perpendicular to a curving direction of the fourth joint 62D has to be input to the instruction input unit 46.

However, as the operator who performs a treatment viewing the observation image uses the camera coordinate system as a reference, the operator may issue an instruction without considering the rolling amount of the manipulator 50 relative to the endoscope 10. That is, the operator may issue an instruction without considering the relation between the camera coordinate system and the manipulator coordinate system. For example, suppose that the manipulator 50 is curved in the X-direction of the camera coordinate system in the condition of FIG. 11B. In this case, the operator may input, to the instruction input unit 46, an instruction to curve the fourth joint 62D in the a-direction of the manipulator coordinate without considering the rolling amount of the manipulator 50 relative to the endoscope 10. However, in the condition of FIG. 11B, the distal end portion of the manipulator 50 is rolled substantially 90° relative to the endoscope 10 in the direction around the axis from the unrolled condition of FIG. 11A. Thus, an instruction of curving in the a-direction of the manipulator coordinate system is input so that the manipulator 50 is curved in a Y-direction (direction substantially perpendicular to the X-direction) of the camera coordinate system. That is, the manipulator 50 is curved in a direction different from a direction intended by the operator.

Therefore, in an endoscopic device according to the present embodiment, the input instruction converter 92 converts the operator input instruction in accordance with the rolling amount of the distal end portion of the manipulator 50 relative to the endoscope 10 in the direction around the axis calculated by the roll information calculator 86. As a result, the operator can manipulate the manipulator 50 without considering the rolling amount of the manipulator 50 relative to the endoscope 10. That is, the operator can manipulate the manipulator 50 on the basis of the camera coordinate system without considering the relation between the camera coordinate system and the manipulator coordinate system.

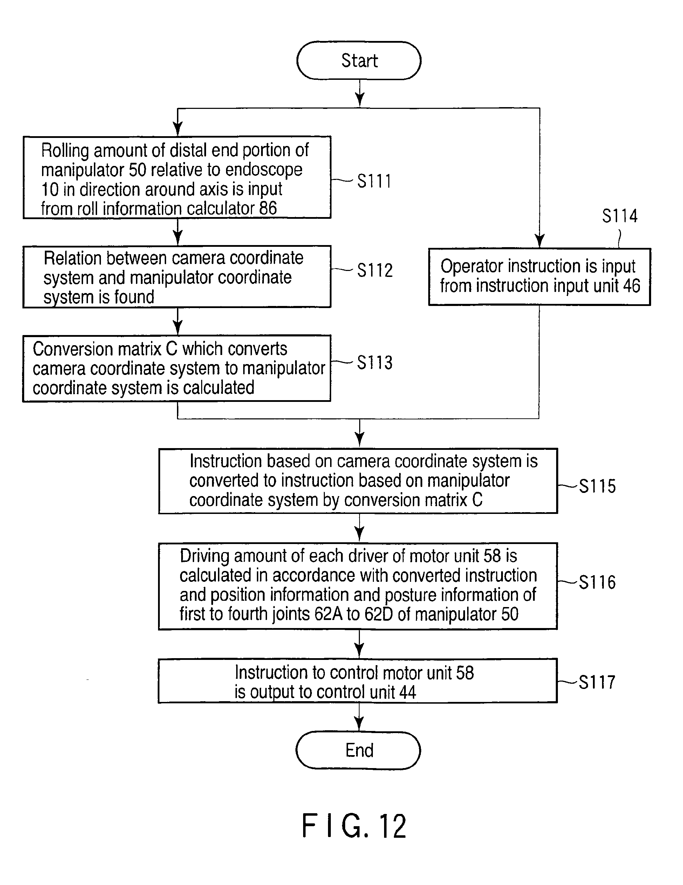

FIG. 12 is a flowchart showing a method of converting the operator input instruction by the input instruction converter 92. As shown in FIG. 12, a rolling amount of the distal end portion of the manipulator 50 relative to the endoscope 10 in the direction around the axis is input to the input instruction converter 92 from the roll information calculator 86 (step S111). In accordance with the input rolling amount of the distal end portion of the manipulator 50 relative to the endoscope 10, the relation between the camera coordinate system and the manipulator coordinate system is found (step S112). Further, a conversion matrix C which converts the camera coordinate system to the manipulator coordinate system is calculated (step S113). Here, the conversion matrix C changes with the relation between the camera coordinate system and the manipulator coordinate system.

Furthermore, an instruction from the operator is input to the input instruction converter 92 from the instruction input unit 46 (step S114). In this case, the instruction from the operator is input on the basis of the camera coordinate system. For example, the operator inputs, to the instruction input unit 46, an instruction to curve the distal end portion of the manipulator 50 in the X-direction of the camera coordinate system in FIG. 11A and FIG. 11B.

Using the conversion matrix C calculated in step S113, the instruction from the operator is converted. The instruction based on the camera coordinate system is converted to an instruction based on the manipulator coordinate system by the conversion matrix C (step S115). For example, suppose that an instruction to curve the distal end portion of the manipulator 50 in the X-direction of the camera coordinate system is input to the instruction input unit 46 in each of the conditions of FIG. 11A and FIG. 11B. In this case, in the condition of FIG. 11A, the instruction is converted to an instruction to curve the distal end portion of the manipulator 50 in the a-direction of the manipulator coordinate system. In the condition of FIG. 11B, the instruction is converted to an instruction to curve the distal end portion of the manipulator 50 in the b-direction of the manipulator coordinate system.

Furthermore, a driving amount of each driver of a motor unit 58 is calculated in accordance with the converted instruction and position information and posture information of the first to fourth joints 62A to 62D of the manipulator 50 (step S116). Here, the position information and posture information of the first to fourth joints 62A to 62D are detected by the calculation unit 90 in accordance with a detection result in each encoder (not shown) of the motor unit 58 and in accordance with, for example, the rolling amount of the distal end portion of the manipulator 50 relative to the endoscope 10. In accordance with the calculated result in step S116, an instruction to control the motor unit 58 is output to the control unit 44 (step S117). For example, suppose that the distal end portion of the manipulator 50 is curved in the X-direction of the camera coordinate system in each of the conditions of FIG. 11A and FIG. 11B. In this case, in the condition of FIG. 11A, the motor unit 58 is driven and controlled to curve, for example, the fourth joint 62D in the a-direction of the manipulator coordinate system. In the condition of FIG. 11B, the motor unit 58 is driven and controlled to curve, in the b-direction of the manipulator coordinate system, for example, the third joint 62C which curves in a direction substantially perpendicular to the curving direction of the fourth joint 62D.

Now, the function of the endoscopic device according to the present embodiment is described. When the operator performs a treatment using the endoscopic device, the operator inputs an instruction based on the camera coordinate system to the instruction input unit 46, and manipulates the manipulator 50. In this case, the input instruction converter 92 of the calculation unit 90 finds the relation between the camera coordinate system and the manipulator coordinate system in accordance with the rolling amount of the distal end portion of the manipulator 50 relative to the endoscope 10 in the direction around the axis calculated by the roll information calculator 86. The input instruction converter 92 then converts the instruction from the operator based on the camera coordinate system to an instruction based on the manipulator coordinate system in accordance with the relation between the camera coordinate system and the manipulator coordinate system. The control unit 44 drives and controls the motor unit 58 in accordance with the instruction converted by the input instruction converter 92. As a result, the operator can manipulate the manipulator 50 without considering the rolling amount of the manipulator 50 relative to the endoscope 10. That is, the operator can manipulate the manipulator 50 on the basis of the camera coordinate system without considering the relation between the camera coordinate system and the manipulator coordinate system.

Thus, the endoscopic device having the above-described configuration has the following advantages. That is, in the endoscopic device according to the present embodiment, the third curving piece 60C of the manipulator curving portion 54 of the manipulator 50 is provided with the marking portions 70A to 70D. On the observation image, the dimensions of at least one of the marking portions 70A to 70D in the direction around the axis of the manipulator 50 can be recognized. When the manipulator 50 is in an unrolled condition and not rolled relative to the endoscope 10 in the direction around the axis, the operator recognizes the position of each of the marking portions 70A to 70D relative to the endoscope 10 in the direction around the axis. Thus, in accordance with the information of the marking portions 70A to 70D on the observation image and the position information of the marking portions 70A to 70D in the unrolled condition, the operator can recognize the rough rolling amount of the distal end portion of the manipulator 50 relative to the endoscope 10 in the direction around the axis.

Furthermore, in the endoscopic device according to the present embodiment, an image signal is input to the distortion remover 80 of the calculation unit 90 from an image processor 34. The distortion remover 80 removes distortion from the observation image on the basis of the distortion information from the memory 84. The Hough transformer 82 then subjects the distortion-free observation image to Hough transformation. As a result of the Hough transformation, the position and posture of at least one of the marking portions 70A to 70D on the observation image are recognized. Information of the marking color of at least one of the marking portions 70A to 70D and its position and posture on the observation image extracted by the Hough transformation is input to the roll information calculator 86. On the basis of the information input from the Hough transformer 82 and the known information recorded in the memory 84, the roll information calculator 86 calculates a rolling amount of the distal end portion of the manipulator 50 relative to the endoscope 10 in the direction around the axis. In this way, the rolling amount of the distal end portion of the manipulator 50 in the direction around the axis can be accurately calculated without providing a detector at the distal end portion of the manipulator 50.

Moreover, in the endoscopic device according to the present embodiment, the operator inputs an instruction based on the camera coordinate system to the instruction input unit 46, and manipulates the manipulator 50. In this case, the input instruction converter 92 of the calculation unit 90 finds the relation between the camera coordinate system and the manipulator coordinate system in accordance with the rolling amount of the distal end portion of the manipulator 50 relative to the endoscope 10 in the direction around the axis calculated by the roll information calculator 86. The input instruction converter 92 then converts the instruction from the operator based on the camera coordinate system to an instruction based on the manipulator coordinate system in accordance with the relation between the camera coordinate system and the manipulator coordinate system. The control unit 44 drives and controls the motor unit 58 in accordance with the instruction converted by the input instruction converter 92. As a result, the operator can manipulate the manipulator 50 without considering the rolling amount of the manipulator 50 relative to the endoscope 10. That is, the operator can manipulate the manipulator 50 on the basis of the camera coordinate system without considering the relation between the camera coordinate system and the manipulator coordinate system.

### (Modifications)

Although the marking portions 70A to 70D are provided in the third curving piece 60C in the two embodiments described above, the marking portions 70A to 70D may be provided in the first curving piece 60A or the second curving piece 60B or may be provided in the grip portion 52. Marking portions may also be provided in multiple parts of the first to third curving pieces 60A to 60C and the grip portion 52. That is, the marking portions have only to be provided with an imaging range of the imaging element 30 and recognizable on the observation screen.

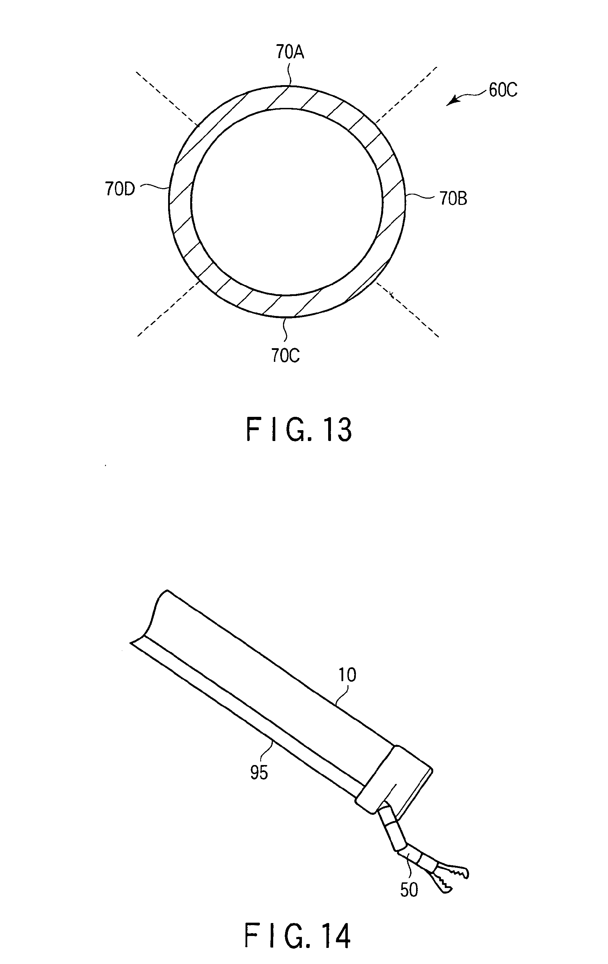

Although the four marking portions 70A to 70D that are provided with different colors are provided apart from one another in the direction around the axis in the embodiments described above, the marking portions are not limited to such a form. For example, as shown in FIG. 13, the four marking portions 70A to 70D that are provided with different colors may not be provided in a state that they are not apart from one another in the direction around the axis. In this case, each of the marking portions 70A to 70D occupies a range of about 90° in the direction around the axis of the third curving piece 60C. Moreover, the above-mentioned colors are not exclusively assigned to the marking portions 70A to 70D. However, it is preferable not to use a color that is the same as or similar to the manipulator 50, and a color that is the same as or similar to blood. Further, instead of assigning different colors to the marking portions 70A to 70D, different pattern designs may be respectively assigned to the marking portions 70A to 70D. In this case, the marking portions 70A to 70D are belt-shaped portions which extend in the longitudinal direction and which are provided with pattern designs different from one another. Still further, the number of the marking portions is not limited to four. However, when the positions and postures of the marking portions on the observation image are extracted by the Hough transformer 82, the shape of at least one of the marking portions has to be recognizable on the observation screen regardless of, for example, the angle of view of the imaging element 30, the positional relation between the imaging element 30 and the manipulator 50, the curving motion of the manipulator 50, and the rolling motion of the manipulator 50.

Although the positions and postures of the belt-shaped marking portions 70A to 70D on the observation image are extracted by the Hough transformer 82 in the embodiments described above, the marking extractor which extracts the positions and postures of the marking portions 70A to 70D on the observation image is not limited to the Hough transformer 82.

Although the manipulator 50 is configured to grip a tissue by the grip portion 52 in the embodiments described above, the manipulator 50 is not limited to this configuration. For example, instead of the grip portion 52, a treatment portion that performs an ultrasonic treatment may be provided. Multiple treatment tool channels 42 may be provided in the endoscope 10, and a treatment may be given by multiple manipulators.

Although the treatment tool channel 42 through which the manipulator 50 is inserted is provided in the endoscope 10 in the embodiments described above, the present invention is not limited to this. For example, as shown in FIG. 14, the endoscopic device may include a treatment tool tube 95 separate from the endoscope 10. In this case, the treatment tool tube 95 is provided with a treatment tool channel, and the manipulator 50 is inserted through the treatment tool channel.

Incidentally, the present invention is not limited to the above embodiments and various modifications can naturally be made without deviating from the spirit and scope of the present invention.

## Claims

1. An endoscopic device comprising:
an endoscope (10) provided with a distal hard portion (14) on a most distal direction side thereof;
a manipulator (50) which is configured to be inserted through an insertion passage (42) provided in the endoscope (10) or in a tube (95) separated from the endoscope (10);
an imaging element (30) which is provided in the distal hard portion (14) of the endoscope (10) and which is configured to image a subject and a distal end portion of the manipulator (50); and
a marking portion (70A-70D) provided at the distal end portion of the manipulator (50) and within an imaging range of the imaging element (30), the position of the marking portion (70A-70D) changing in a direction around an axis in response to a rolling motion of the manipulator (50) relative to the endoscope (10).

2. The endoscopic device according to claim 1, further comprising:
an image processor (34) which is configured to process an image signal of an observation image captured by the imaging element (30); and
a calculation unit (38, 90) which is configured to calculate a rolling amount of the manipulator (50) relative to the endoscope (10) in the direction around the axis on the basis of the image signal from the image processor (34).

3. The endoscopic device according to claim 2, wherein
the calculation unit (38, 90) includes
a recorder (84) in which known information is recorded,
a distortion remover (80) which is configured to remove distortion from the observation image,
a marking extractor (82) which is configured to extract position information and posture information of the marking portion (70A-70D) on the distortion-free observation image, and
a roll information calculator (86) which is configured to calculate the rolling amount of the manipulator (50) relative to the endoscope (10) in the direction around the axis on the basis of the extracted position information and posture information of the marking portion (70A-70D) on the observation image and the known information recorded in the recorder (84).

4. The endoscopic device according to claim 3, further comprising:
an instruction input unit (46) which configured to input an instruction based on a camera coordinate system having its origin in the imaging element (30) on the observation image to manipulate the manipulator (50),
wherein the calculation unit (90) includes an input instruction converter (92), the input instruction converter (92) being configured to find a relation between the camera coordinate system and a manipulator coordinate system on the basis of the rolling amount of the manipulator (50) relative to the endoscope (10) calculated by the roll information calculator (86), and being configured to convert the instruction based on the camera coordinate system from the instruction input unit (46) to an instruction based on the manipulator coordinate system in accordance with the relation between the camera coordinate system and the manipulator coordinate system, and the manipulator coordinate system having its origin in a distal end of the manipulator (50) on the observation image.

5. The endoscopic device according to claim 1, wherein the marking portion (70A-70D) includes belt-shaped portions (70A-70D) which extend in a longitudinal direction and which are provided with colors different from that of the manipulator (50) and different from one another.

6. The endoscopic device according to claim 1, wherein the marking portion (70A-70D) includes belt-shaped portions (70A-70D) which extend in a longitudinal direction and which are provided with pattern designs different from one another.
